# FASCICULE DE BREVET EUROPEEN

(11) **EP 3 129 085 B1**
(45) Date de publication et mention de la délivrance du brevet: **18.07.2018**
(21) Numéro de dépôt: 15725716.3
(22) Date de dépôt: 09.04.2015
(51) Int. Cl.: A61M 5/50, A61M 5/315, A61M 5/31, A61M 5/32, A61M 5/20

(54) **AUTOINJECTEUR**
AUTOINJEKTOR
AUTOINJECTOR

(30) Priorité: 11.04.2014 FR 1453228
(43) Date de publication de la demande: 15.02.2017
(73) Titulaire: APTAR France SAS, 27110 Le Neubourg (FR)
(72) Inventeur: SAUSSAYE, Anthony, 27400 LOUVIERS (FR); HIS, Olivier, 27430 Saint Etienne Du Vauvray (FR)
(74) Mandataire: CAPRI
(86) Numéro de dépôt international: PCT/FR2015/050934
(87) Numéro de publication internationale: WO 2015/155482

(56) Documents cités:
- EP-A1- 2 716 318
- WO-A1-2012/045350
- WO-A1-2012/072568
- WO-A2-2005/097238
- WO-A2-2014/053378
- US-A1- 2009 270 804

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. En particulier, il est important que l'utilisateur puisse aisément et clairement identifier si l'autoinjecteur est prêt pour être utilisé ou au contraire s'il a déjà été utilisé. Cette information peut être cruciale en cas de crise. Par ailleurs, la plupart de ces autoinjecteurs étant à usage unique, le coût de fabrication et d'assemblage est également un facteur dont il faut tenir compte.

Les documents WO 2005/097238 et WO 2012/045350 décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui informe clairement l'utilisateur que l'autoinjecteur a déjà été utilisé.

La présente invention a aussi pour but de fournir un autoinjecteur qui soit simple et peu coûteux à fabriquer et à assembler.

La présente invention a donc pour objet un autoinjecteur comportant un corps adapté à recevoir un réservoir, ledit réservoir contenant du produit fluide et comportant un piston et une aiguille, tel qu'une seringue pré-remplie, ledit autoinjecteur comportant un manchon actionneur comportant une extrémité de contact destinée à venir en contact avec le corps de l'utilisateur, ledit manchon actionneur s'étendant au moins partiellement à l'intérieur dudit corps et étant déplaçable par rapport audit corps entre des positions projetées, dans lesquelles ledit manchon actionneur fait au moins partiellement saillie hors dudit corps, et une position d'actionnement, dans laquelle ledit manchon actionneur est axialement déplacé vers l'intérieur dudit corps, ledit manchon actionneur étant dans une première position projetée avant actionnement de l'autoinjecteur et dans une seconde position projetée après actionnement de l'autoinjecteur, ledit manchon actionneur, dans ladite seconde position projetée, s'étendant davantage axialement hors dudit corps que dans ladite première position projetée, ledit manchon actionneur comportant une zone annulaire qui, dans ladite première position projetée, est disposée à l'intérieur dudit corps et qui, dans ladite seconde position projetée, est disposée à l'extérieur dudit corps, ladite zone annulaire comportant un indicateur visuel pour indiquer à l'utilisateur que l'autoinjecteur a été actionné, et ledit manchon actionneur comportant, axialement adjacent à ladite zone annulaire, un épaulement radialement saillant vers l'extérieur, ledit épaulement se projetant radialement vers l'extérieur par rapport audit indicateur visuel, protégeant ainsi ledit indicateur visuel contre tout contact avec la surface intérieure du corps lors des déplacements du manchon actionneur dans ledit corps, l'extrémité inférieure du manchon actionneur, qui vient en contact avec la zone où l'injection a lieu, comportant également un épaulement radial saillant vers l'extérieur.

Avantageusement, ledit épaulement est formé par une projection radiale périphérique.

En variante, ledit épaulement est formé par deux ou plusieurs projections radiales réparties autour de la périphérie dudit manchon actionneur.

Avantageusement, ledit indicateur visuel est formé par une étiquette autocollante apposée la surface externe dudit manchon actionneur, au moins sur ladite zone annulaire.

Avantageusement, ledit manchon actionneur, dans ladite seconde position projetée, est verrouillé en position par des pattes, solidaires du corps ou du réservoir, et coopérant avec des ouvertures dudit manchon actionneur.

Avantageusement, ledit autoinjecteur comportant une tige de piston adaptée à coopérer avec le piston dudit réservoir, ladite tige de piston étant déplaçable entre une position de repos et une position d'injection dans laquelle ladite tige de piston a déplacé le piston du réservoir pour injecter le produit fluide à travers l'aiguille, un ressort d'actionnement étant prévu pour solliciter ladite tige de piston vers sa position d'injection.

Avantageusement, la surface radialement externe dudit épaulement est lisse, pour limiter les éventuels frottements avec la surface interne du corps lors des déplacements dudit manchon actionneur dans ledit corps.

Avantageusement, ledit manchon actionneur comporte en outre un indicateur d'information, visible dans ladite première position projetée du manchon actionneur.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
Les figures 1 et 2 sont des vues de côté d'un autoinjecteur selon un mode de réalisation avantageux, respectivement avant et après injection,
Les figures 3 et 4 sont des vues schématiques en section transversale d'un autoinjecteur selon un mode de réalisation avantageux, respectivement avant et après injection, et
Les figures 5 et 6 sont des vues de détail respectivement des figures 3 et 4.

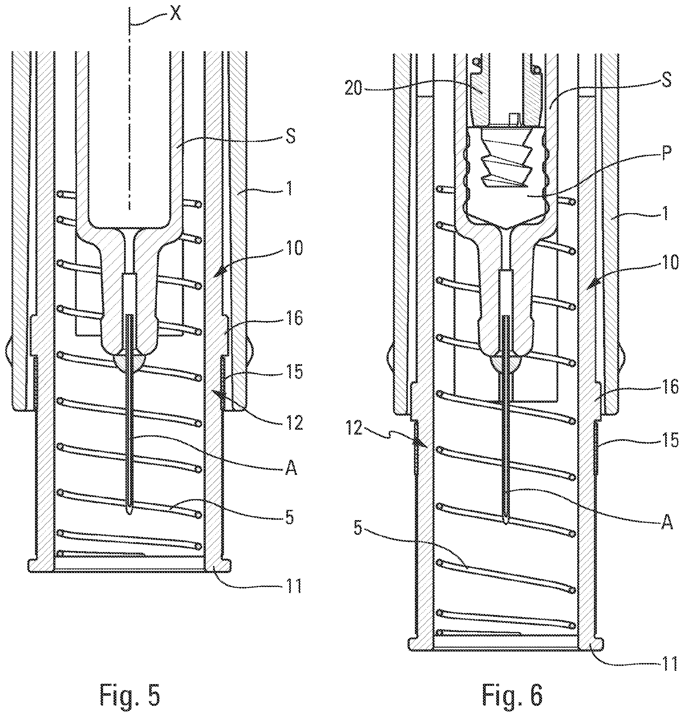

Dans la description ci-après, les termes "supérieur" et "inférieur" se réfèrent aux positions représentés sur les figures. Les termes "axial" et "radial" se réfèrent à l'axe central longitudinal X représenté sur la figure 5

L'autoinjecteur va être décrit ci-après en référence à un mode de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

L'autoinjecteur représenté sur les figures comporte un corps 1 dans lequel coulisse axialement un manchon actionneur 10, dont l'extrémité inférieure 11 est destinée à venir en contact avec le corps du patient autour de la zone d'injection.

Un réservoir S peut être inséré dans ledit autoinjecteur. Ce réservoir S contient du produit fluide, et comporte un piston P et une aiguille A. Le piston P est adapté à se déplacer dans ledit réservoir S pour injecter le produit fluide à travers ladite aiguille A.

La présente description sera faite en référence à une seringue S, qui peut être de tout type. Plus généralement, il est entendu que le terme « seringue » dans la présente description englobe tout type de réservoir associé à une aiguille. De préférence, le réservoir S est une seringue pré-remplie.

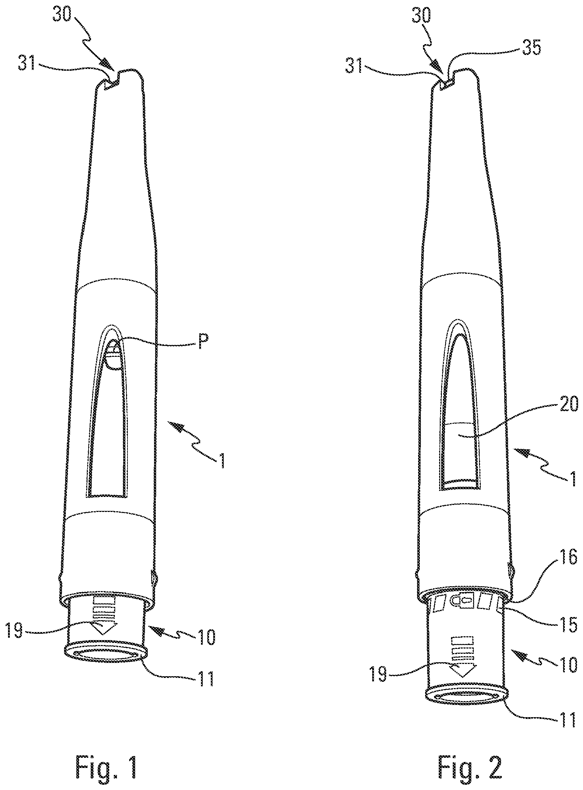

Avant actionnement, le manchon actionneur 10 est dans une première position projetée dans laquelle il entoure l'aiguille A, comme représenté sur les figures 1, 3 et 5. Lors de l'actionnement, le manchon actionneur 10 coulisse à l'intérieur du corps 1 vers une position d'actionnement, pour exposer l'aiguille A et permettre le piquage puis l'injection du produit fluide. Après l'injection, le manchon actionneur 10 revient dans une seconde position projetée, dans laquelle il est à nouveau disposé autour de l'aiguille A, pour éviter tout risque de blessure avec ladite aiguille, comme représenté sur les figures 2, 4 et 6. Le manchon actionneur 10 est avantageusement sollicité vers ses positions projetées par un ressort 5, qui peut être de type quelconque.

L'autoinjecteur comporte également un système d'injection automatique, comportant notamment une tige de piston 20 adaptée à coopérer avec le piston P pour le déplacer dans le réservoir S pour distribuer le produit fluide à travers l'aiguille A. Cette tige de piston 20 est classiquement sollicitée par un ressort 25 vers sa position de distribution, et retenue avant actionnement dans sa position de repos par une serrure d'injection appropriée. Un dispositif d'indication visuelle et/ou sonore 30 peut être prévu à l'extrémité supérieure du corps 1 de l'autoinjecteur, avec une ou plusieurs fenêtres 31 adaptée à recevoir un indicateur 35 après injection, visible à travers la ou les fenêtre(s) 31. Ces différents modules ou systèmes (système d'injection automatique, serrure d'injection, dispositif d'indication visuelle et/ou sonore), qui ne sont pas essentiels au fonctionnement de la présente invention, ne seront pas décrits plus en détail ci-après. Ils pourraient être réalisés différemment du mode de réalisation particulier représenté sur les dessins. En particulier, ils pourraient être réalisés conformément aux enseignements des documents WO 2013/175148 ou US2014088505.

Selon l'invention, ledit manchon actionneur 10, lorsqu'il est dans sa seconde position projetée, après injection, s'étend davantage axialement hors dudit corps 1 que dans ladite première position projetée, avant actionnement. Ainsi, ledit manchon actionneur 10 comporte une zone annulaire 12 qui, dans ladite première position projetée, est disposée à l'intérieur dudit corps 1 et qui, dans ladite seconde position projetée, est disposée à l'extérieur dudit corps 1. Cette zone annulaire 12 comporte un indicateur visuel 15, tel qu'un marquage spécifique, qui informe l'utilisateur que l'autoinjecteur a déjà été utilisé. Cet indicateur visuel 15 peut être quelconque, par exemple en couleur, et/ou comporter des symboles, des lettres ou des mots, ou tout autre marquage aisément identifiable par l'utilisateur. Cet indicateur visuel 15 peut être apposé sur ladite zone annulaire 12 par tout moyen approprié, par exemple via une étiquette autocollante collée sur la surface externe du manchon actionneur 10. D'autres moyens pour réaliser ledit indicateur visuel 15 sont envisageables.

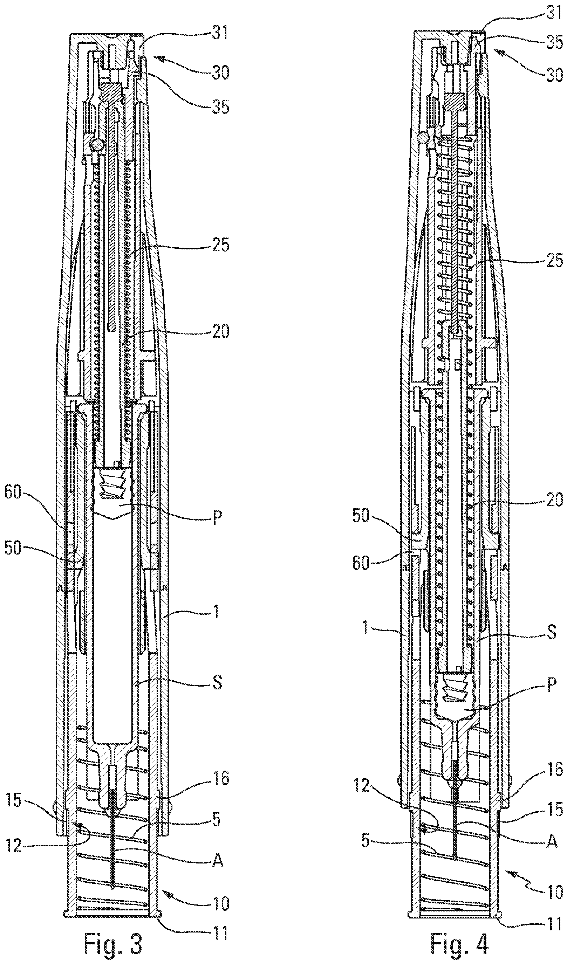

Avantageusement, dans ladite seconde position projetée après injection, ledit manchon actionneur 10 est verrouillé, et ne peut plus être déplacé axialement vers l'intérieur dudit corps 1. Ce verrouillage peut par exemple être réalisé par des pattes 50, solidaires du corps 1 ou du réservoir S, et coopérant avec des ouvertures 60 dudit manchon actionneur 10 lorsque celui-ci atteint sa seconde position projetée. Dans l'exemple des figures 3 et 4, les pattes 50 sont solidaires du réservoir S. L'indicateur visuel 15 peut alors comporter des moyens identifiant un tel verrouillage, par exemple un cadenas dans l'exemple de la figure 2.

Selon un aspect de l'invention, ledit manchon actionneur 10 comporte, axialement adjacent à ladite zone annulaire 12 en direction dudit corps 1, un épaulement 16 radialement saillant vers l'extérieur. Cet épaulement 16 est avantageusement entièrement à l'intérieur dudit corps 1 dans ladite première position projetée du manchon actionneur, avant injection, comme clairement visible sur les figures 3 et 5. Dans ladite seconde position projetée, après injection, ledit épaulement 16 est de préférence aussi au moins partiellement à l'intérieur dudit corps 1, comme représenté sur les figures 2, 4 et 6. Cet épaulement 16 peut être une projection radiale périphérique, ou être formé par deux ou plusieurs projections radiales réparties autour de la périphérie dudit manchon actionneur 10. Cet épaulement 16 se projette donc radialement vers l'extérieur par rapport à ladite zone annulaire 12, et donc par rapport audit indicateur visuel 15. De cette manière, ledit indicateur visuel 15 est protégé contre tout contact avec la surface intérieure du corps 1 lors des déplacements du manchon actionneur 10 dans ledit corps 1, d'abord de sa première position projetée vers sa position d'actionnement, puis de sa position d'actionnement vers sa seconde position projetée. En l'absence de l'épaulement 16, ce déplacement en va-et-vient du manchon actionneur 10 dans le corps 1 pourrait par frottements altérer ledit indicateur visuel 15, notamment lorsque celui-ci est formé par une étiquette collée sur la surface externe du manchon actionneur 10. La présente invention garantit donc une indication parfaitement fiable de l'utilisateur après utilisation de l'autoinjecteur. De préférence, la surface radialement externe dudit épaulement 16 est lisse, pour limiter les éventuels frottements avec la surface interne du corps 1.

Comme représenté sur les dessins, l'extrémité inférieure 11 du manchon actionneur 10, qui vient en contact avec la zone où l'injection a lieu, comporte également un épaulement radial saillant vers l'extérieur. Cette mise en oeuvre améliore la protection de l'indicateur visuel et permet notamment d'apposer aisément une étiquette autocollante sur la surface externe cylindrique dudit manchon actionneur 10, entre ledit épaulement 16 et ledit épaulement formé par ladite extrémité inférieure 11. Cette étiquette peut alors comporter ledit indicateur visuel 15 au niveau de ladite zone annulaire 12 qui n'est visible que dans la seconde position projetée. Avantageusement, cette étiquette peut en outre comporter un indicateur d'information 19, visible dans ladite première position projetée, avant injection, tel que par exemple la flèche représentée sur la figure 1, qui indique à l'utilisateur dans quel sens il faut appuyer pour actionner l'autoinjecteur.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à un mode de réalisation avantageux, il est entendu que diverses modifications sont possibles pour l'homme du métier sans sortir du cadre de la présente invention tel que défini par les revendications annexées.

## Revendications

1. Autoinjecteur comportant un corps (1) adapté à recevoir un réservoir (S), ledit réservoir (S) contenant du produit fluide et comportant un piston (P) et une aiguille (A), tel qu'une seringue pré-remplie, ledit autoinjecteur comportant un manchon actionneur (10) comportant une extrémité de contact (11) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur, **caractérisé en ce que**:
- ledit manchon actionneur (10), dans ladite seconde position projetée, s'étend davantage axialement hors dudit corps (1) que dans ladite première position projetée, ledit manchon actionneur (10) comportant une zone annulaire (12) qui, dans ladite première position projetée, est disposée à l'intérieur dudit corps (1) et qui, dans ladite seconde position projetée, est disposée à l'extérieur dudit corps (1), ladite zone annulaire (12) comportant un indicateur visuel (15) pour indiquer à l'utilisateur que l'autoinjecteur a été actionné,
- et **en ce que** ledit manchon actionneur (10) comporte, axialement adjacent à ladite zone annulaire (12), un épaulement (16) radialement saillant vers l'extérieur, ledit épaulement (16) se projetant radialement vers l'extérieur par rapport audit indicateur visuel (15), protégeant ainsi ledit indicateur visuel (15) contre tout contact avec la surface intérieure du corps (1) lors des déplacements du manchon actionneur (10) dans ledit corps (1), l'extrémité inférieure (11) du manchon actionneur (10), qui vient en contact avec la zone où l'injection a lieu, comportant également un épaulement radial saillant vers l'extérieur

2. Autoinjecteur selon la revendication 1, dans lequel ledit épaulement (16) est formé par une projection radiale périphérique.

3. Autoinjecteur selon la revendication 1, dans lequel ledit épaulement (16) est formé par deux ou plusieurs projections radiales réparties autour de la périphérie dudit manchon actionneur (10).

4. Autoinjecteur selon l'une quelconque des revendications précédentes, dans lequel ledit indicateur visuel (15) est formé par une étiquette autocollante apposée la surface externe dudit manchon actionneur (10), au moins sur ladite zone annulaire (12).

5. Autoinjecteur selon l'une quelconque des revendications précédentes, dans lequel ledit manchon actionneur (10), dans ladite seconde position projetée, est verrouillé en position par des pattes (50), solidaires du corps (1) ou du réservoir (S), et coopérant avec des ouvertures (60) dudit manchon actionneur (10).

6. Autoinjecteur selon l'une quelconque des revendications précédentes, dans lequel ledit autoinjecteur comportant une tige de piston (20) adaptée à coopérer avec le piston (P) dudit réservoir (S), ladite tige de piston (20) étant déplaçable entre une position de repos et une position d'injection dans laquelle ladite tige de piston (20) a déplacé le piston (P) du réservoir (S) pour injecter le produit fluide à travers l'aiguille (A), un ressort d'actionnement (25) étant prévu pour solliciter ladite tige de piston (20) vers sa position d'injection.

7. Autoinjecteur selon l'une quelconque des revendications précédentes, dans lequel la surface radialement externe dudit épaulement (16) est lisse, pour limiter les éventuels frottements avec la surface interne du corps (1) lors des déplacements dudit manchon actionneur (10) dans ledit corps (1).

8. Autoinjecteur selon l'une quelconque des revendications précédentes, dans lequel ledit manchon actionneur (10) comporte en outre un indicateur d'information (19), visible dans ladite première position projetée du manchon actionneur (10).

## Patentansprüche

1. Autoinjektor, aufweisend einen Körper (1), der dazu geeignet ist, einen Behälter (S) aufzunehmen, wobei der Behälter (S) ein fluides Produkt enthält und einen Kolben (P) und eine Nadel (A), wie eine vorgefüllte Spritze, aufweist, wobei der Autoinjektor eine Betätigungsmuffe (10) aufweist, die ein Kontaktende (11) aufweist, das dazu gedacht ist, mit dem Körper des Benutzers in Kontakt zu kommen, wobei sich die Betätigungsmuffe (10) zumindest teilweise ins Innere des Körpers (1) erstreckt und in Bezug auf den Körper (1) zwischen vorstehenden Positionen, in denen die Betätigungsmuffe (10) zumindest teilweise aus dem Körper (1) vorsteht, und einer Betätigungsposition verschiebbar ist, in der die Betätigungsmuffe (10) zum Inneren des Körpers (1) axial verschoben ist, wobei sich die Betätigungsmuffe (10) vor der Betätigung des Autoinjektors in einer ersten vorstehenden Position und nach der Betätigung des Autoinjektors in einer zweiten vorstehenden Position befindet, **dadurch gekennzeichnet, dass**:
- sich die Betätigungsmuffe (10) in der zweiten vorstehenden Position weiter axial außerhalb des Körpers (1) erstreckt als in der ersten vorstehenden Position, wobei die Betätigungsmuffe (10) einen ringförmigen Bereich (12) aufweist, der in der ersten vorstehenden Position im Inneren des Körpers (1) angeordnet ist und der in der zweiten vorstehenden Position außerhalb des Körpers (1) angeordnet ist, wobei der ringförmige Bereich (12) einen Sichtanzeiger (15) aufweist, um dem Benutzer anzuzeigen, dass der Autoinjektor betätigt worden ist,
- und die Betätigungsmuffe (10) axial benachbart zum ringförmigen Bereich (12) einen Absatz (16) aufweist, der radial nach außen vorspringt, wobei sich der Absatz (16) radial nach außen in Bezug auf den Sichtanzeiger (15) erstreckt, wodurch er den Sichtanzeiger (15) vor jeglichem Kontakt mit der Innenfläche des Körpers (1) bei Verschiebungen der Betätigungsmuffe (10) in dem Körper (1) schützt, wobei das untere Ende (11) der Betätigungsmuffe (10), das mit dem Bereich, in dem die Injektion stattfindet, in Kontakt kommt, auch einen radial nach außen vorstehenden Absatz aufweist.

2. Autoinjektor nach Anspruch 1, wobei der Absatz (16) durch einen radialen umfänglichen Vorsprung gebildet ist.

3. Autoinjektor nach Anspruch 1, wobei der Absatz (16) durch zwei oder mehrere radiale Vorsprünge gebildet ist, die um den Umfang der Betätigungsmuffe (10) verteilt sind.

4. Autoinjektor nach einem der vorhergehenden Ansprüche, wobei der Sichtanzeiger (15) durch ein selbstklebendes Etikett gebildet ist, das auf der Außenfläche der Betätigungsmuffe (10), zumindest auf dem ringförmigen Bereich (12), angebracht ist.

5. Autoinjektor nach einem der vorhergehenden Ansprüche, wobei die Betätigungsmuffe (10) in der zweiten vorstehenden Position durch Ansätze (50), die mit dem Körper (1) oder dem Behälter (S) einstückig gebildet sind und mit Öffnungen (60) der Betätigungsmuffe (10) zusammenwirken, verriegelt wird.

6. Autoinjektor nach einem der vorhergehenden Ansprüche, wobei der Autoinjektor eine Kolbenstange (20) aufweist, die dazu geeignet ist, mit dem Kolben (P) des Behälters (S) zusammenzuwirken, wobei die Kolbenstange (20) zwischen einer Ruheposition und einer Injektionsposition verschiebbar ist, in der die Kolbenstange (20) den Kolben (P) des Behälters (S) verschoben hat, um das fluide Produkt durch die Nadel (A) zu injizieren, wobei eine Betätigungsfeder (25) vorgesehen ist, um die Kolbenstange (20) in ihre Injektionsposition vorzuspannen.

7. Autoinjektor nach einem der vorhergehenden Ansprüche, wobei die radial äußere Oberfläche des Absatzes (16) glatt ist, um mögliche Reibungen mit der Innenfläche des Körpers (1) bei Verschiebungen der Betätigungsmuffe (10) in dem Körper (1) zu begrenzen.

8. Autoinjektor nach einem der vorhergehenden Ansprüche, wobei die Betätigungsmuffe (10) des Weiteren einen Informationsanzeiger (19) aufweist, der in der ersten vorstehenden Position der Betätigungsmuffe (10) sichtbar ist.

## Claims

1. An autoinjector comprising a body (1) that is adapted to receive a reservoir (S), said reservoir (S) containing fluid and including a piston (P) and a needle (A), such as a pre-filled syringe, said autoinjector further comprising an actuator sleeve (10) that includes a contact end (11) for coming into contact with the user's body, said actuator sleeve (10) extending inside said body (1) at least in part, and being movable relative to said body (1) between projecting positions in which said actuator sleeve (10) projects out from said body (1) at least in part, and an actuated position in which said actuator sleeve (10) is moved axially into said body (1), said actuator sleeve (10) being in a first projecting position before actuation of the autoinjector, and in a second projecting position after actuation of the autoinjector, the autoinjector being **characterized in that**:
• said actuator sleeve (10), in said second projecting position, extends axially further out from said body (1) than in said first projecting position, said actuator sleeve (10) including an annular zone (12) that, in said first projecting position, is arranged inside said body (1) and that, in said second projecting position, is arranged outside said body (1), said annular zone (12) including a visual indicator (15) for indicating to the user that the autoinjector has been actuated;
• and **in that**, axially adjacent to said annular zone (12), said actuator sleeve (10) includes an outwardly-projecting radial shoulder (16), said shoulder (16) projecting radially outwards relative to said visual indicator (15), thereby protecting said visual indicator (15) against any contact with the inside surface of the body (1) while the actuator sleeve (10) is moving in said body (1), the bottom end (11) of the actuator sleeve (10), that comes into contact with the zone in which injection takes place, also including an outwardly-projecting radial shoulder.

2. An autoinjector according to claim 1, wherein said shoulder (16) is formed by a peripheral radial projection.

3. An autoinjector according to claim 1, wherein said shoulder (16) is formed by two or more radial projections that are distributed around the periphery of said actuator sleeve (10).

4. An autoinjector according to any preceding claim, wherein said visual indicator (15) is formed by a self-adhesive label that is applied to the outside surface of said actuator sleeve (10), at least to said annular zone (12).

5. An autoinjector according to any preceding claim, wherein, in said second projecting position, said actuator sleeve (10) is locked in position by tabs (50) that are secured to the body (1) or to the reservoir (S), and that co-operate with openings (60) in said actuator sleeve (10).

6. An autoinjector according to any preceding claim, wherein said autoinjector includes a piston rod (20) that is adapted to co-operate with the piston (P) of said reservoir (S), said piston rod (20) being movable between a rest position and an injection position in which said piston rod (20) has moved the piston (P) of the reservoir (S) so as to inject the fluid through the needle (A), an actuator spring (25) being provided so as to urge said piston rod (20) towards its injection position.

7. An autoinjector according to any preceding claim, wherein the radially-outer surface of said shoulder (16) is smooth, so as to limit possible friction with the inside surface of the body (1) while said actuator sleeve (10) is moving in said body (1).

8. An autoinjector according to any preceding claim, wherein said actuator sleeve (10) further includes an information indicator (19) that can be seen in said first projecting position of the actuator sleeve (10).
